# FASCICULE DE BREVET EUROPEEN

(11) **EP 4 267 215 B1**
(45) Date de publication et mention de la délivrance du brevet: **20.05.2026**
(21) Numéro de dépôt: 21839517.6
(22) Date de dépôt: 17.12.2021
(51) Int. Cl.: A61M 1/36, A61M 1/38, A61M 1/02, B01D 39/14

(54) **UNITÉ DE FILTRATION DES LEUCOCYTES À ADHÉSION DES PLAQUETTES RÉDUITE**
LUEKOZYTENFILTEREINHEIT MIT GERINGERER PLÄTTCHENADHÄSION
LEUKOCYTE FILTERING UNIT WITH REDUCED PLATELET ADHESION

(30) Priorité: 22.12.2020 FR 2013941
(43) Date de publication de la demande: 01.11.2023
(73) Titulaire: Maco Pharma, 59420 Mouvaux (FR)
(72) Inventeur: DUCOROY, Laurent, 59890 Quesnoy-sur-Deule (FR)
(74) Mandataire: Sayettat, Julien Christian
(86) Numéro de dépôt international: PCT/EP2021/086371
(87) Numéro de publication internationale: WO 2022/136138

(56) Documents cités:
- EP-A1- 2 783 717
- JP-A- H05 262 656

## Description

L'invention concerne une unité de filtration destinée à permettre la déleucocytation sélective d'un fluide contenant des plaquettes sanguines, ainsi qu'un système à poches comprenant une telle unité.

Elle s'applique typiquement à la filtration du sang ou d'un composant sanguin contenant des plaquettes tel qu'un plasma riche en plaquettes (PRP), un concentré plaquettaire unitaire (CPU) ou un assemblage de concentrés plaquettaires (CP).

Le sang total est constitué de deux types de composants : les cellules sanguines comprenant les globules rouges, les leucocytes et les plaquettes, et le plasma, liquide jaune pâle dans lequel les cellules sanguines sont en suspension.

Actuellement, on ne transfuse aux patients que les composants sanguins nécessaires à leur état. Par exemple, on ne transfuse que des concentrés plaquettaires aux patients atteints de thrombocytopénie, c'est-à-dire dont la teneur en plaquettes dans le sang est réduite.

Il s'est avéré que les leucocytes ont des effets indésirables très importants, ce qui a conduit à chercher à les éliminer des composants sanguins destinés à la transfusion. En effet, les leucocytes augmentent le risque de rejet immunitaire tel que la maladie du greffon contre l'hôte et favorisent la transmission d'agents infectieux. Il a aussi été démontré que les leucocytes affectaient de façon négative la conservation des plaquettes.

Pour éliminer les leucocytes des composants sanguins destinés à la transfusion, on connaît déjà des unités de filtration renfermant un milieu de déleucocytation. Dans de telles unités, le milieu de déleucocytation comprend une ou plusieurs membrane(s) et/ ou une ou plusieurs couche(s) de non-tissé réalisée(s) en un matériau polymère et traitée(s) de façon à améliorer le taux de déleucocytation, la récupération des composants sanguins, le temps d'amorçage de la filtration et/ou la sélectivité de la filtration, par exemple en laissant passer les plaquettes.

Pour éliminer les leucocytes tout en laissant passer les plaquettes, plusieurs traitements de surface polymères de milieux de déleucocytation ont déjà été proposés.

Par exemple, le document US 4 936 998 décrit un milieu filtrant pour éliminer de façon sélective les leucocytes. Le milieu filtrant est composé de fibres revêtues d'un polymère contenant des groupes hydrophiles non ioniques et des groupes fonctionnels basiques contenant un azote. Un polymère particulier est un copolymère de méthacrylate d'hydroxyéthyle et de méthacrylate de diéthylaminoéthyle.

Le document EP-2 783 717 divulgue également un filtre permettant la déleucocytation sélective d'un fluide comprenant des plaquettes, ledit filtre comprenant un milieu poreux enduit d'un polymère comportant une chaîne principale hydrophobe et une chaîne pendante hydrophile de poly(oxyde d'éthylène). Par exemple, le polymère est obtenu par copolymérisation d'un macromonomère de méthacrylate de poly(oxyde d'éthylène) et d'un monomère de méthacrylate de méthyle.

Un autre filtre pour éliminer les leucocytes est divulgué dans le document US 2006/0207937. Le filtre comprend un matériau support hydrophobe et un matériau de revêtement polymère obtenu par polyréaction d'un mélange de monomères hydrophobes et hydrophiles, tel qu'un mélange d'acétate de vinyle et de pyrrolidone de vinyle. Le mélange comprend entre 60 à 99% de monomère hydrophobe.

Le document EP-1 452 193 propose également un filtre pour éliminer de façon sélective les leucocytes tout en minimisant la perte en plaquettes, ledit filtre comprenant un polymère obtenu à partir de : (i) un (méth)acrylate d'hydroxyalkyle, (ii) un monomère contenant des groupes azotes basiques et (iii) un monomère comprenant des chaînes d'oxyde d'éthylène contenant entre 2 et 9 répétitions d'oxyde d'éthylène. La masse moléculaire moyenne en masse du polymère est supérieure à 100 000 pour éviter les problèmes d'élution.

Le document JP H05 262656 divulgue un filtre pour éliminer de façon sélective les leucocytes d'un composant plaquettaire, la surface dudit filtre étant revêtue d'un polymère de (méth)acrylate d'alcoxyalkyle. Le (méth)acrylate d'alcoxyalkyle peut être copolymérisé avec un monomère de (méth)acrylate d'alkyle. Dans les exemples, une membrane poreuse de polyuréthane est greffée avec un polymère d'acrylate de méthoxyéthyle ou d'acrylate de méthoxybutyle.

Enfin, le document EP 1 156 067 propose un matériau de filtre capable d'éliminer les leucocytes et les plaquettes du sang tout en laissant passer les globules rouges. Ce matériau de filtre est revêtu d'un copolymère de constitué d'un monomère de (méth)acrylate d'alcoxyalkyle et d'un monomère ayant un groupe fonctionnel basique tel qu'un (méth)acrylate d'aminoalkyle ou un (méth)acrylamide d'aminoalkyle.

La demanderesse a développé un matériau de filtre revêtu d'un copolymère constitué d'un monomère de (méth)acrylate d'alcoxyalkyle, mais qui, contrairement au matériau de filtre du document EP 1 156 067, est capable de retenir les leucocytes tout en laissant passer les plaquettes.

Selon un premier aspect, l'invention propose une unité de filtration destinée à permettre la déleucocytation sélective d'un fluide contenant des plaquettes sanguines comprenant une enveloppe extérieure munie d'au moins un orifice d'entrée et d'au moins un orifice de sortie, l'enveloppe renfermant un élément poreux interposé entre lesdits orifices, ledit élément poreux renfermant un milieu de déleucocytation imprégné d'un polymère, ledit polymère ayant la formule générale -(A)m-(B)n- (I), dans laquelle A est une unité provenant d'un monomère d'acrylate ou de méthacrylate d'alcoxyalkyle, B est une unité provenant d'un monomère polymérisable hydrophobe, et m et n sont des entiers dont la somme m+n est égale à 100, m étant un entier supérieur à 80.

Selon un second aspect, l'invention concerne un système à poches pour la déleucocytation d'un fluide contenant des plaquettes sanguines, comprenant :
- une unité de filtration selon le premier aspect de l'invention, et
- une poche de recueil du filtrat, ladite poche étant reliée, par l'intermédiaire d'une tubulure, à un orifice de sortie de l'unité de filtration.

Selon un troisième aspect, l'invention porte sur un ensemble comprenant un système à poches selon le deuxième aspect de l'invention dans lequel l'unité de filtration comprend une enveloppe souple et un étui rigide destiné à contenir ladite unité de filtration, ledit étui étant configuré pour empêcher le gonflement de l'unité de filtration lors de la filtration.

Les dessins annexés illustrent l'invention :
[Fig.1] représente une vue schématique d'une unité de filtration selon l'invention.
[Fig.2] représente une vue schématique en coupe d'une unité de filtration de la [Fig.1].
[Fig.3] représente une vue schématique d'un équipement permettant de réaliser l'imprégnation d'un milieu de déleucocytation selon le principe de foulardage séchage.
[Fig.4] représente une vue schématique d'un système à poches selon l'invention.
[Fig.5] représente une vue schématique d'un étui pour l'unité de filtration de la [Fig.1].
[Fig.6] représente une vue schématique de l'étui de la [Fig.5] contenant l'unité de filtration de la [Fig.1].

L'invention propose une unité de filtration destinée à permettre la déleucocytation sélective d'un fluide contenant des plaquettes sanguines.

Un fluide contenant des plaquettes est par exemple un sang total, un plasma riche en plaquettes (PRP), un concentré plaquettaire unitaire (CPU) ou un assemblage de concentrés plaquettaires (CP).

Notamment, l'unité de filtration est destinée à filtrer un concentré plaquettaire obtenu par aphérèse ou à partir d'une ou plusieurs unités de sang total.

Il existe plusieurs méthodes de préparation des concentrés plaquettaires issus de sang total, parmi lesquelles on peut citer la méthode de préparation à partir de couches leucoplaquettaires (European Directorate for the Quality of Medicines. Healthcare (EDQM), Council of Europe: Guide to the preparation, use and quality assurance of blood components, (2019)).

Selon cette méthode de préparation, une unité de sang total est centrifugée de sorte à séparer le sang en trois couches : une couche de surnageant de plasma, une couche intermédiaire leucoplaquettaire comprenant un mélange de leucocytes, plaquettes, globules rouges et plasma, et une couche de culot de globules rouges. La couche leucoplaquettaire est séparée des autres couches. Entre quatre et huit couches leucoplaquettaires ainsi séparées, sont ensuite réunies et re-suspendues dans du plasma ou dans une solution additive pour plaquettes, telle que la solution SSP+ (Macopharma). Ce mélange est à nouveau centrifugé (centrifugation douce) de sorte à obtenir une couche de surnageant de concentré plaquettaire et une couche de culot de globules rouges. Le concentré plaquettaire est ensuite séparé et conservé jusqu'à 4 à 7 jours à température ambiante.

Lorsqu'on utilise une solution additive, le concentré plaquettaire final est constitué en général des plaquettes re-suspendues dans un mélange constitué de plasma (20-40%) et de solution additive (60-80%).

Lors de la préparation, lorsqu'on utilise un assemblage de quatre à six couches leucoplaquettaires, on obtient un concentré plaquettaire dit « simple dose ». Lorsqu'on utilise un assemblage de sept ou huit couches leucoplaquettaires, on obtient un concentré plaquettaire dit « double dose ».

Il existe ainsi une diversité de concentrés plaquettaires selon le nombre de couches leucoplaquettaires utilisé lors de sa préparation et la présence ou non d'une solution additive pour plaquettes.

L'unité de filtration selon l'invention présente l'avantage de pouvoir filtrer des concentrés plaquettaires de tout type en obtenant des résultats conformes aux normes en vigueur, en terme de taux de récupération des plaquettes et de nombre de leucocytes résiduels.

L'unité de filtration permet une déleucocytation sélective, c'est-à-dire qu'elle est capable de retenir les leucocytes tout en laissant passer les plaquettes.

Notamment, lorsque le fluide à filtrer est un concentré plaquettaire issu de sang total, l'unité de filtration est apte à obtenir un nombre de leucocytes dans le fluide filtré qui est inférieur à 1.10⁶ et à laisser passer au moins 70 % des plaquettes sanguines. Le nombre de plaquettes dans le concentré plaquettaire après filtration est notamment d'au moins 2.10¹¹ plaquettes.

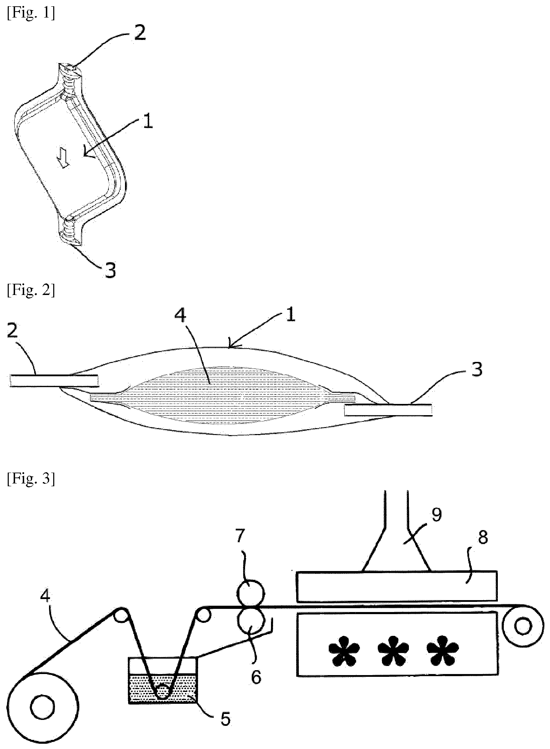

Comme représenté sur les figures 1 et 2, l'unité de filtration 1 comprend une enveloppe extérieure munie d'au moins un orifice d'entrée 2 et d'au moins un orifice de sortie 3, l'enveloppe renfermant un élément poreux 4 interposé entre lesdits orifices 2,3, ledit élément poreux 4 renfermant un milieu de déleucocytation imprégné d'un polymère.

L'enveloppe extérieure de l'unité de filtration est souple, rigide ou semi-rigide.

Le milieu de déleucocytation imprégné d'un polymère est apte à retenir les leucocytes par adsorption et/ou par filtration des leucocytes présents dans le fluide contenant les plaquettes. Le milieu de déleucocytation imprégné empêche également les plaquettes d'adhérer à sa surface.

Dans un milieu de déleucocytation, les leucocytes sont retenus par l'un et/ou l'autre des mécanismes suivants. Le premier mécanisme est l'adsorption des leucocytes à la surface du milieu. Les leucocytes sont adsorbés sur des surfaces cationiques et moyennement hydrophobes. La qualité de l'adsorption dépend également de la surface de contact disponible pour l'adsorption des leucocytes. Le deuxième mécanisme est le tamisage et dépend principalement du diamètre des pores du milieu de déleucocytation.

D'autre part, pour une filtration sélective, la surface du milieu de déleucocytation doit être est suffisamment biocompatible pour que les plaquettes n'adhèrent pas à sa surface.

Le milieu de déleucocytation de l'unité de filtration de l'invention est imprégné d'un polymère, ledit polymère ayant la formule générale -(A)m-(B)n- (I), dans laquelle A est une unité provenant d'un monomère d'acrylate d'alcoxyalkyle ou de méthacrylate d'alcoxyalkyle, B est une unité provenant d'un monomère polymérisable hydrophobe, et m et n sont des entiers dont la somme m+n est égale à 100, m étant un entier supérieur à 80.Ce polymère est aussi appelé polymère d'imprégnation.

Ce polymère possède la particularité d'être constitué d'une unité provenant d'un monomère de (méth)acrylate d'alcoxyalkyle et notamment d'acrylate de méthoxyéthyle qui possède une bonne propriété biocompatible, c'est-à-dire que les plaquettes mais aussi les leucocytes ne s'adsorbent pas à surface.

Notamment, l'unité A possède la structure suivante : 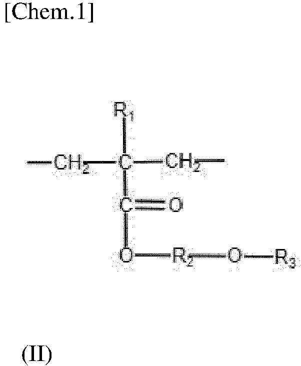 dans laquelle R1 est choisi parmi le groupe constitué d'un atome d'hydrogène ou le groupe méthyle, R2 est une liaison alkylène en C1-C4, linéaire ou ramifiée, et R3 est un groupement alkyle en C1-C4, linéaire ou ramifié.

Afin de favoriser l'adhésion des leucocytes, le polymère est également constitué d'une unité B provenant d'un monomère polymérisable hydrophobe.

Avantageusement, le monomère polymérisable hydrophobe est choisi parmi le groupe constitué d'un acrylate d'alkyle, un méthacrylate d'alkyle et un alkylate de vinyle. Par exemple, ce monomère est le méthacrylate de méthyle ou l'acétate de vinyle.

Ce monomère hydrophobe contribue également en outre au maintien du polymère à la surface du milieu de déleucocytation.

En particulier, l'unité B du polymère d'imprégnation ne contient pas de groupe azote basique. Notamment, l'unité B ne provient pas d'un monomère de (méth)acrylate d'alkylaminoalkyle ou d'acrylamide d'alkylaminoalkyle.

L'unité B possède l'une des deux structures suivantes : 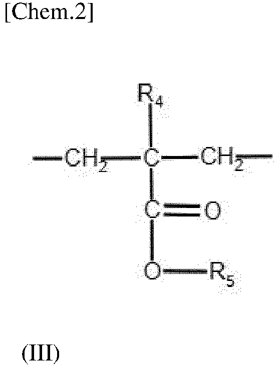 dans laquelle R4 est choisi parmi le groupe constitué d'un atome d'hydrogène et le groupement méthyle et R5 est un groupement alkyle en C1-C6, linéaire ou ramifié, ou

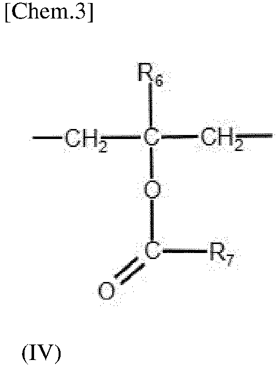

Dans laquelle R6 est choisi parmi le groupe constitué d'un atome d'hydrogène et le groupement méthyle et R7 est un groupement alkyle en C1-C6, linéaire ou ramifié.

Afin de ne pas dégrader la passivation des surfaces vis-à-vis de plaquettes sanguines, la quantité de monomère de (méth) acrylate d'alcoxyalkyle dans le polymère est supérieure à 80% en moles - c'est-à-dire m est supérieur à 80, notamment supérieure à 90% en moles - c'est-à-dire m est supérieur à 90.

En effet, un taux de monomère de (méth) acrylate d'alcoxyalkyle inférieur à 80% en mole dans le polymère d'imprégnation entraîne un taux de récupération des plaquettes plus faibles.

Avantageusement, m est un entier inférieur à 96 afin de retenir suffisamment les leucocytes dans le milieu de déleucocytation.

Encore plus avantageusement, m est un entier compris entre 90 et 96.

Selon l'invention, le polymère possède une masse molaire moyenne en masse (Mw) comprise entre 15 000 g/mol et 115 000 g/mol, en particulier comprise entre 20 000 et 60 000 g/mol.

Ce polymère ayant une molaire moyenne en masse relativement peu élevée contribue à une meilleure solubilisation dans un solvant tel que l'éthanol.

La masse molaire moyenne en masse est déterminée par exemple par chromatographie d'exclusion stérique dans le tétrahydrofurane avec un étalonnage polystyrène.

Le polymère possède une masse molaire moyenne en nombre (Mn) comprise entre 5 000 g/mol et 40 000 g/mol, en particulier comprise entre 6 000 et 12 000 g/mol.

Comme la masse molaire moyenne en masse, la masse molaire moyenne en nombre est déterminée par exemple par chromatographie d'exclusion stérique dans le tétrahydrofurane avec un étalonnage polystyrène.

L'indice de polymolécularité du polymère est compris entre 1 et 6, en particulier entre 1 et 5. L'indice de polymolécularité est le rapport de la masse molaire moyenne en masse (Mw) sur la masse molaire moyenne du polymère en nombre (Mn). Cet indice permet de caractériser globalement la dispersité des masses molaires d'un polymère. Un indice proche de 1 indique que toutes les chaînes molaires d'un polymère sont de même longueur.

Le polymère d'imprégnation ainsi obtenu est avantageusement insoluble dans l'eau, soluble dans un solvant alcoolique ou cétonique et résistant à la stérilisation vapeur.

Pour imprégner le milieu de déleucocytation du polymère d'imprégnation, on prépare d'abord une solution d'imprégnation constituée du polymère d'imprégnation en tant que soluté et d'un liquide organique en tant que solvant.

La solution d'imprégnation contient une faible concentration de polymère d'imprégnation. Notamment, la quantité de polymère dissous dans le solvant est comprise entre 1 et 10 g/L, en particulier comprise 2 et 5 g/L.

Les solvants d'imprégnation sont notamment un solvant alcoolique tel que le méthanol ou l'éthanol, ou un solvant cétonique tel que l'acétone ou la méthyléthylcétone.

Selon une réalisation, l'imprégnation est réalisée selon un principe de foulardage séchage, par exemple avec un équipement représenté sur la [Fig.3]. Le milieu de déleucocytation 4 est trempé dans la solution d'imprégnation 5. Le surplus de solution imprégnée sur le milieu de déleucocytation est ensuite exprimé ou essoré en passant entre deux rouleaux 6,7 dont la pression est comprise entre 1 et 5 bars. Puis le milieu de déleucocytation 4 est convoyé dans un four 8 équipé d'une ventilation mécanique 9 afin de le sécher par évaporation du solvant. La vitesse, comprise entre 1 et 10 m/min, est régulée en fonction de la nature et de la quantité de solvant emportée par le milieu de déleucocytation.

La quantité de polymère déposée sur le milieu de déleucocytation est comprise entre 1 et 10 mg/g de milieu de déleucocytation, en particulier entre 4 et 7 mg/g de milieu de déleucocytation. Cette quantité est par exemple déterminée par d'extraction dans un solvant puis par chromatographie phase liquide.

Selon une réalisation, le milieu de déleucocytation comprend des fibres de polyester, par exemple des fibres de polybutylène téréphtalate ou de polyéthylène téréphtalate.

Notamment, le milieu de déleucocytation est formé d'au moins une couche de non-tissé. En particulier, le milieu de déleucocytation comprend entre 5 et 40 couches de non-tissé, et plus particulièrement entre 15 et 25 couches de non-tissé.

Par exemple, le diamètre des fibres de la ou des couches de non-tissé est compris entre 0,3 et 7 µm, avec une moyenne comprise entre 1 et 3 µm.

Pour retenir mécaniquement les leucocytes, le diamètre moyen des pores de la couche de non-tissé est avantageusement compris entre 3 et 15 µm, notamment entre 7 et 10 µm.

Avec une quantité de polymère déposée sur le milieu de déleucocytation comprise entre 1 et 10 mg/g de milieu de déleucocytation, le diamètre moyen des pores et le diamètre moyen des fibres de la couche de non-tissé avant et après imprégnation demeurent sensiblement les mêmes.

Selon une réalisation particulière, l'élément poreux de l'unité de filtration comprend en outre un pré filtre et/ou post-filtre qui sont réalisés sous la forme d'au moins une couche de non-tissé et qui sont disposés respectivement côté amont et côté aval du milieu de déleucocytation.

Ces pré-filtres ou post-filtres ont notamment une taille de pores moyenne supérieure à celle du milieu de déleucocytation, par exemple comprise entre 25 et 50 µm.

Les pré-filtres ou post-filtres sont imprégnés ou non d'un polymère facilitant le passage des plaquettes.

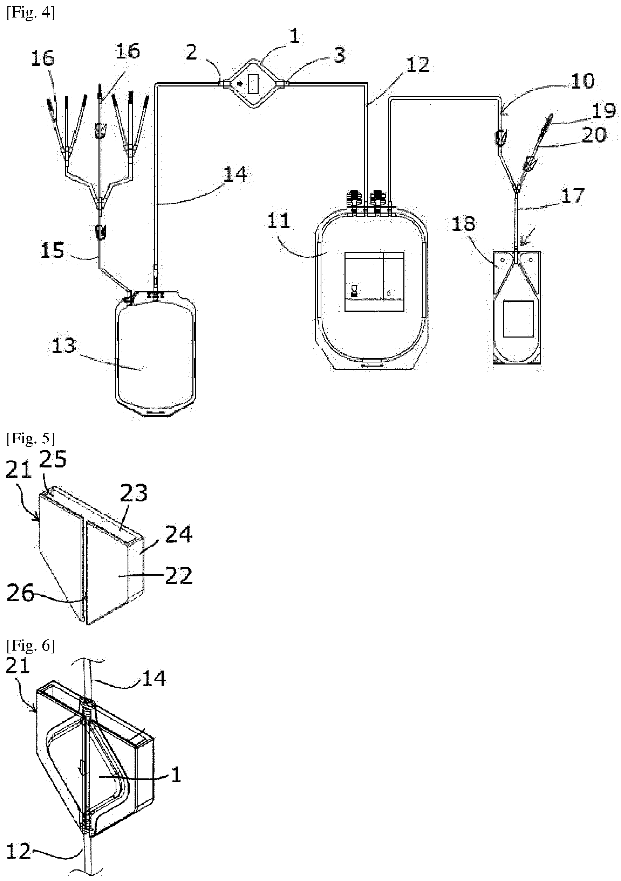

Selon un autre aspect exemplifié sur la [Fig.4], l'invention concerne un système à poches 10 pour la déleucocytation d'un fluide contenant des plaquettes sanguines comprenant :
- une unité de filtration 1 selon le premier aspect de l'invention, et
- une poche de recueil du filtrat 11, ladite poche 11 étant reliée, par l'intermédiaire d'une tubulure 12, à un orifice de sortie 3 de l'unité de filtration 1.

En relation avec la [Fig.4], un système à poches particulier est décrit pour la préparation et la filtration d'un assemblage de couches leucoplaquettaires.

Le système à poches 10 comprend une poche 13 d'assemblage connectée ou destinée à être connectée par l'intermédiaire d'une première tubulure 14 à l'unité de filtration 1 de l'invention. L'unité de filtration 1 est connectée par l'intermédiaire d'une deuxième tubulure 12 à la poche 11 de recueil du filtrat.

Pour la préparation d'un assemblage de couches leucoplaquettaires, la poche 13 d'assemblage est en communication fluidique avec un ensemble de tubulures constitué d'une tubulure principale 15 et de tubulures secondaires 16 branchées sur la tubulure principale. Ces tubulures secondaires 16 sont destinées à être connectées de façon stérile à au moins quatre poches contenant une couche leucoplaquettaire et éventuellement à une poche contenant une solution additive de conservation des plaquettes ou du plasma.

Selon une réalisation particulière, la poche 11 de recueil du filtrat est en communication fluidique par l'intermédiaire d'une troisième tubulure 17 à une poche satellite 18 destinée à recevoir l'air présent dans la poche 11 de recueil du filtrat et/ou au recueil d'un échantillon du fluide contenu dans la poche 11 de recueil du filtrat.

Afin de prélever des échantillons du fluide contenu dans la poche 11, le système à poches comprend une quatrième tubulure 20 branchée sur la troisième tubulure 17, ladite quatrième tubulure 20 étant pourvue à son extrémité d'un moyen de prélèvement 19. L'échantillon prélevé par ce moyen est utilisé pour la détection d'une contamination bactérienne (Système Bact/Alert^{®} par exemple).

Les tubulures sont pourvues de clamp pour contrôler l'écoulement des fluides à l'intérieur du système à poches.

Lorsque l'unité de filtration comprend une enveloppe souple, l'unité de filtration 1 gonfle lors de la filtration. Afin d'éviter ce gonflement, on place avantageusement l'unité de filtration dans un étui rigide 21 tel que celui représenté sur la [Fig.5].

L'invention dans son troisième aspect concerne ainsi un ensemble comprenant d'une part un système à poches 10 selon le deuxième aspect de l'invention dans lequel l'unité de filtration 1 comprend une enveloppe souple et d'autre part un étui rigide 21 destiné à contenir ladite unité de filtration, ledit étui étant configuré pour empêcher le gonflement de l'unité de filtration lors de la filtration.

En lien avec les figures 5 et 6, cet étui rigide 21 comprend une cavité configurée pour réceptionner l'unité de filtration 1.

L'étui 21 comprend une paroi avant 22 et une paroi arrière 23, substantiellement parallèles entre elles, et reliées par deux parois latérales 24,25. La paroi avant 21 comprend une fente 26 suffisamment large pour permettre le passage de la tubulure 12 en aval de l'unité de filtration 1 permettant d'insérer l'unité de filtration 1 dans l'étui. La géométrie de l'étui correspond au moins en partie à la géométrie de l'unité de filtration.

L'étui est réalisé par impression 3D ou pour moulage par injection.

En relation avec le système à poches de la [Fig.4], on décrit maintenant une utilisation particulière du système à poches 10 pour la préparation d'un concentré plaquettaire simple dose en solution additive.

Entre quatre et six poches contenant une couche leucoplaquettaire sont connectées de façon stérile, par exemple à l'aide d'un appareil de connexion stérile de type SCD (Terumo) aux tubulures secondaires 16 d'un système à poches 10. Une poche de solution de conservation de type SSP+ (Macopharma, France) est également connectée de façon stérile à l'une des tubulures secondaires 16.

Les couches leucoplaquettaires s'écoulent dans la poche 13 d'assemblage. Les poches ayant contenues lesdites couches leucoplaquettaires sont ensuite rincées à l'aide de la solution de conservation. Puis on transfère le reste de la solution de conservation vers la poche 13 d'assemblage. L'ensemble de tubulures 15,16 est ensuite séparé de la poche 13 d'assemblage par soudure.

La poche 13 d'assemblage est centrifugée pour obtenir une couche sédimentaire de globules rouges et une couche de surnageant de concentré plaquettaire.

La poche 13 d'assemblage contenant le culot de globules rouges et le surnageant de concentré plaquettaire est ensuite pressée, par exemple à l'aide d'un dispositif de presse de type Macopress Smart (Macopharma, France) afin d'envoyer le concentré plaquettaire dans la poche 11 de recueil du filtrat via l'unité de filtration 1 de l'invention et d'obtenir un concentré de plaquettes déleucocyté.

Lors de cette étape de séparation sur presse et filtration, l'unité de filtration est placée dans un étui 21 tel que celui représenté sur les figures 5 et 6 afin d'éviter le gonflement de l'unité de filtration lors du passage du fluide à filtrer.

### Exemple 1 : Polymère de poly(méthacrylate de méthyle-co-acrylate de 2-méthoxyéthyle) (P1 : p(MMA10-MEA90))

Dans un réacteur avec un agitateur magnétique, on mélange sous atmosphère inerte 7,7 g de méthacrylate de méthyle (0.077 mol) et 117,13 g d'acrylate de méthoxyéthyle (0,9 mol) avec 305 g de tétrahydrofurane et 76 g d'éthanol. Le mélange est agité sous barbotage avec un gaz inerte à température ambiante pendant 20 minutes.

2,5 g d'azobisisobutyronitrile (AIBN) est ensuite ajouté et le mélange est encore agité pendant 10 minutes sous atmosphère inerte.

Le mélange est ensuite porté dans un bain d'huile à 70°C pendant 20 heures puis les solvants sont évaporés à 40°C. Le polymère est enfin lavé et séché.

Le polymère P1 obtenu est non soluble dans l'eau. Sa masse molaire en nombre (Mn) est d'environ 6 700 g/mol, son indice de polymolécularité est de 2,6. Le polymère comprend 10% en moles de méthacrylate de méthyle.

### Exemple 2 : Polymère de poly(méthacrylate de méthyle-co-acrylate de 2-méthoxyéthyle) (P2 : p(MMA15-MEA85))

Le mode de préparation de l'exemple 1 a été adapté pour obtenir un poly(méthacrylate de méthyle-co- acrylate de 2-méthoxyéthyle) ayant une masse molaire en nombre (Mn) d'environ 5 800 g/mol, un indice de polymolécularité de 2,9. Le polymère comprend 15% en moles de méthacrylate de méthyle.

### Exemple 3 :Polymère de poly(acétate de vinyle-co-acrylate de 2-méthoxyéthyle) (P3 :p(VAc9-MEA91))

Dans un réacteur, on mélange 647 g d'éthylméthylcétone et 163 g d'éthanol absolu sous atmosphère inerte et barbotage avec un gaz inerte.

On ajoute ensuite 1,36 g de 2,2'-azobis(2,4-dimethylvaleronitrile), 16,7 g d'acétate de vinyle et 187 g d'acrylate de 2-méthoxyéthyle. Le mélange est chauffé à 60°C pendant 20 heures ; puis le polymère est purifié par distillation directe ou par lavage à l'eau.

Le copolymère P3 obtenu est non soluble dans l'eau. Sa masse molaire en masse (Mw) est d'environ 35 700 g/mol, son indice de polymolécularité est de 4,3. Le polymère comprend 9% en moles d'acétate de vinyle.

### Exemple 4 :Polymère de poly(acétate de vinyle-co-acrylate de 2-méthoxyéthyle) (P4 :p(VAc5-MEA95))

Le même mode de préparation a été utilisé pour obtenir un poly(acétate de vinyle-co-acrylate de 2-méthoxyéthyle) ayant une masse molaire en nombre (Mn) d'environ 7 300 g/mol, un indice de polymolécularité de 5,3 et 5% en moles d'acétate de vinyle.

### Exemple 5 : Préparation des unités de filtration

Des couches de non-tissé de polybutylène téréphtalate (42 g/m², diamètre moyen de pores de 8,5 µm) ont été imprégnées selon le principe du foulardage séchage décrit cidessus avec des solutions d'imprégnation obtenues en solubilisant le polymère dans de l'éthanol. La solution d'imprégnation comprend une concentration d'environ 3 à 4 g/l de polymère d'imprégnation.

### Exemple 6 : Filtration d'un concentré plaquettaire en solution additive - simple dose

Des concentrés plaquettaires ont été préparés à partir d'un assemblage de quatre couches leucoplaquettaires et de 300 ml de solution additive SSP+ (Macopharma).

La poche contenant les quatre couches leucoplaquettaires et la solution SSP+ est centrifugée afin d'obtenir un surnageant de concentré plaquettaire.

La séparation et la filtration sous pression du concentré plaquettaire est réalisée avec une presse automatique. La séparation/filtration est arrêtée lorsque le culot de globules rouges atteint la moitié de tubulure aval de l'unité de filtration.

Les résultats sont présentés dans le tableau ci-dessous. Le temps de filtration est inférieur à 2 minutes.

Le taux moyen de récupération des plaquettes est déterminée par rapport à la quantité de plaquettes initialement présente dans le surnageant de concentré plaquettaire avant filtration. Celle-ci est calculée en soustrayant le nombre de plaquettes des couches leucoplaquettaires du nombre de plaquettes restant dans le culot de globules rouges après filtration/séparation.

**[Tableaux1]**

| Polymère | P1 | P2 | P3 | P4 |
|---|---|---|---|---|
| Nombre de couches dans l'unité de filtration | 20 | 20 | 20 | 20 |
| Nombre de filtration | 10 | 14 | 15 | 14 |
| Nombre moyen de après filtration (10⁹/U) | 259 ± 36 | 246 ± 29 | 249 ± 29 | 264 ± 46 |
| Taux moyen de récupération des plaquettes (%) | 78,1 ± 10,7 | 70,7 ± 7,4 | 77,4 ± 5,7 | 78,3 ± 7,9 |
| Nombre moyen de leucocytes après filtration (10⁶/U) | 0,032 ± 0,036 | 0,014 ± 0,023 | 0,019 ± 0,023 | 0,022 ± 0,004 |

### Exemple 7 : Filtration d'un concentré plaquettaire en solution additive - double dose

Des concentrés plaquettaires ont été préparés à partir de huit couches leucoplaquettaires et 280 ml de solution SSP+ à l'aide d'un système à poches comme celui représenté sur la [Fig.3].

L'unité de filtration comprend une enveloppe souple renfermant entre 18 et 24 couches de non-tissé de polybutylène téréphthalate imprégnées du polymère P3 à une concentration de 4g/L.

Pour la filtration, l'unité de filtration est insérée dans un étui rigide comme celui représenté sur la [Fig.5].

La séparation et la filtration des concentrés plaquettaires sont réalisées avec une presse automatique (Macopress EVO Smart, Macopharma). On arrête la séparation/ filtration lorsque le culot de globules rouges atteint la moitié de la tubulure en aval de l'unité de filtration.

Les résultats sont montrés dans le tableau ci-après. Le temps de filtration est inférieur à cinq minutes.

Le taux moyen de récupération des plaquettes est calculé par rapport à la quantité de plaquettes initialement présente dans le surnageant de concentré plaquettaire avant filtration.

**[Tableaux2]**

| | | | | |
|---|---|---|---|---|
| Nombre de couches dans l'unité de filtration | 18 | 20 | 22 | 24 |
| Nombre de filtration | 5 | 5 | 5 | 5 |
| Nombre moyen de plaquettes après filtration (10⁹/U) | 577 ± 46 | 575 ± 55 | 591 ± 57 | 598 ± 60 |
| Taux minimal de récupération des plaquettes (%) | 98 | 98 | 94 | 94 |
| Nombre moyen de leucocytes après filtration (10⁶/U) | 1,14 ± 1,93 | 0,40 ± 1,33 | 0,58 ± 0,8 | 0,30 ± 0,28 |

### Exemple 8 : Filtration d'un concentré plaquettaire en plasma - simple dose

Des concentrés plaquettaires ont été préparés à partir de cinq couches leucoplaquettaires et une unité de plasma à l'aide d'un système à poches comme celui représenté sur la [Fig.2].

L'unité de filtration comprend une enveloppe souple renfermant 24 couches de non-tissé de polybutylène téréphtalate imprégnées du polymère P3 à une concentration de 3,25 g/L).

Pour la filtration sous pression, l'unité de filtration est insérée dans un étui rigide comme celui représenté sur la [Fig.5] afin d'éviter le gonflement de l'unité de filtration.

La séparation et la filtration des concentrés plaquettaires sont réalisées avec une presse automatique. On arrête la séparation/filtration lorsque le culot de globules rouges atteint la moitié de la tubulure en aval de l'unité de filtration.

Les résultats sont montrés dans le tableau ci-après. Les temps de filtration sont inférieurs à 3 minutes.

Le taux moyen de récupération des plaquettes est calculé par rapport à la quantité de plaquettes initialement présente dans le surnageant de concentré plaquettaire avant filtration.

**[Tableaux3]**

| | |
|---|---|
| Nombre de filtration | 16 |
| Nombre moyen de plaquettes après filtration (10⁹/U) | 361 ± 38 |
| Taux moyen de récupération des plaquettes (%) | 91,4 ± 4,1 |
| Nombre moyen de leucocytes après filtration (10⁶/U) | 0,157 ± 0,166 |

## Revendications

1. Unité de filtration (1) destinée à permettre la déleucocytation sélective d'un fluide contenant des plaquettes sanguines comprenant une enveloppe extérieure munie d'au moins un orifice d'entrée (2) et d'au moins un orifice de sortie (3), l'enveloppe renfermant un élément poreux (4) interposé entre lesdits orifices, ledit élément poreux renfermant un milieu de déleucocytation imprégné d'un copolymère, ladite unité de filtration étant **caractérisée en ce que** ledit copolymère a la formule générale -(A)m-(B)n-(I), dans laquelle A est une unité provenant d'un monomère d'acrylate d'alcoxyalkyle ou de méthacrylate d'alcoxyalkyle, B est une unité provenant d'un monomère polymérisable hydrophobe, et m et n sont des entiers dont la somme m+n est égale à 100, m étant un entier supérieur à 80.

2. Unité de filtration selon la revendication 1, **caractérisé en ce que** l'unité A possède la structure suivante : 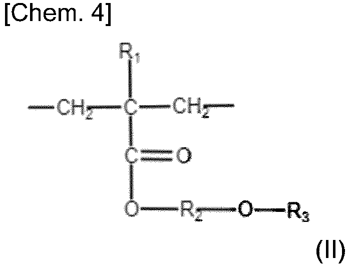 dans laquelle R1 est choisi parmi le groupe constitué d'un atome d'hydrogène ou le groupe méthyle, R2 est une liaison alkylène en C1-C4, linéaire ou ramifiée, et R3 est un groupement alkyle en C1-C4, linéaire ou ramifié.

3. Unité de filtration selon la revendication 1 ou 2, **caractérisée en ce que** l'unité A provient de l'acrylate de méthoxyéthyle.

4. Unité de filtration selon l'une quelconque des revendications 1 à 3, **caractérisée en ce que** m est un entier compris entre 90 et 96.

5. Unité de filtration selon l'une quelconque des revendications 1 à 4, **caractérisée en ce que** B provient d'un monomère choisi parmi le groupe constitué d'un acrylate d'alkyle, un méthacrylate d'alkyle et un alkylate de vinyle.

6. Unité de filtration selon l'une quelconque des revendications 1 à 5, **caractérisé en ce que** l'unité B possède l'une des deux structures suivantes : 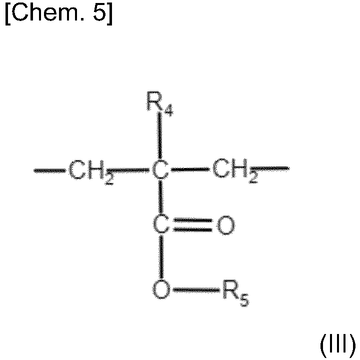 dans laquelle R4 est choisi parmi le groupe constitué d'un atome d'hydrogène et le groupement méthyle et R5 est un groupement alkyle en C1-C6, linéaire ou ramifié, ou 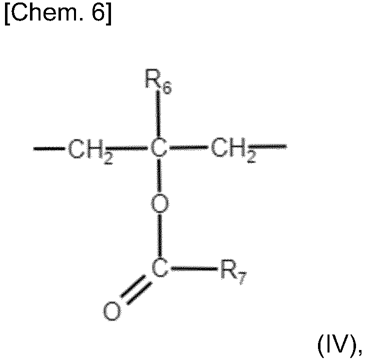 dans laquelle R6 est choisi parmi le groupe constitué d'un atome d'hydrogène et le groupement méthyle et R7 est un groupement alkyle en C1-C6.

7. Unité de filtration selon l'une quelconque des revendications 1 à 6, **caractérisée en ce que** l'unité B provient du méthacrylate de méthyle ou de l'acétate de vinyle.

8. Unité de filtration selon l'une quelconque des revendications 1 à 7, **caractérisée en ce que** la masse molaire moyenne en nombre dudit polymère est comprise entre 5 000 g/mol et 40 000 g/mol.

9. Unité de filtration selon l'une quelconque des revendications 1 à 8, **caractérisée en ce que** la masse molaire moyenne en masse dudit copolymère est comprise entre 15 000 g/mol et 115 000 g/mol.

10. Unité de filtration selon l'une quelconque des revendications 1 à 9, **caractérisée en ce que** le polymère possède un indice de polymolécularité compris entre 1 et 6.

11. Unité de filtration selon l'une quelconque des revendications 1 à 10, **caractérisée en ce que** la quantité de copolymère déposée sur le milieu de déleucocytation est comprise entre 1 et 10 mg/g de milieu de déleucocytation.

12. Unité de filtration selon l'une quelconque des revendications 1 à 11, **caractérisée en ce que** le milieu de déleucocytation comprend des fibres de polybutylène téréphtalate ou de polyéthylène téréphtalate.

13. Unité de filtration selon l'une quelconque des revendications 1 à 12 **caractérisée en ce que** le milieu de déleucocytation est formé d'au moins une couche de non-tissé.

14. Unité de filtration selon la revendication 13, **caractérisée en ce que** le diamètre moyen des pores de la couche de non-tissé est compris entre 3 et 15 µm.

15. Système à poches (10) pour la déleucocytation d'un fluide contenant des plaquettes sanguines, **caractérisé en ce qu'**il comprend :
- une unité de filtration selon l'une quelconque des revendications 1 à 14, et
- une poche (11) de recueil du filtrat, ladite poche étant reliée, par l'intermédiaire d'une tubulure (12), à un orifice de sortie (3) de l'unité de filtration (1).

16. Ensemble comprenant d'un part un système à poches (10) selon la revendication 15 dans lequel l'unité de filtration (1) comprend une enveloppe souple et d'autre part un étui rigide (21) destiné à contenir de ladite unité de filtration, ledit étui (21) étant configuré pour empêcher le gonflement de l'unité de filtration (1) lors de la filtration.

## Patentansprüche

1. Filtereinheit (1), die dazu bestimmt ist, die selektive Leukozytendepletion eines Fluids zu ermöglichen, das Blutplättchen enthält, umfassend eine äußere Hülle, die mit mindestens einer Einlassöffnung (2) und mindestens einer Auslassöffnung (3) versehen ist, wobei die Hülle ein poröses Element (4) umschließt, das zwischen die Öffnungen eingefügt ist, das poröse Element ein Leukozytendepletionsmedium umschließt, das mit einem Copolymer imprägniert ist, wobei die Filtereinheit **dadurch gekennzeichnet ist, dass** das Copolymer die allgemeine Formel -(A)m-(B)n-(I) aufweist, worin A eine Einheit ist, die von einem Alkoxyalkylacrylat- oder Alkoxyalkylmethacrylatmonomer stammt, B eine Einheit ist, die von einem hydrophoben polymerisierbaren Monomer stammt, und m und n ganze Zahlen sind, deren Summe m+n gleich 100 ist, wobei m eine ganze Zahl größer als 80 ist.

2. Filtereinheit nach Anspruch 1, **dadurch gekennzeichnet, dass** die Einheit A die folgende Struktur aufweist: 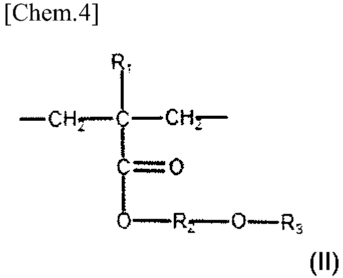 wobei R1 ausgewählt ist aus der Gruppe, bestehend aus einem Wasserstoffatom oder einer Methylgruppe, R2 eine lineare oder verzweigte C1-C4-Alkylenbindung ist und R3 eine lineare oder verzweigte C1-C4-Alkylgruppierung ist.

3. Filtereinheit nach Anspruch 1 oder 2, **dadurch gekennzeichnet, dass** die Einheit A von dem Methoxyethylacrylat stammt.

4. Filtereinheit nach einem der Ansprüche 1 bis 3, **dadurch gekennzeichnet, dass** m eine ganze Zahl zwischen 90 und 96 ist.

5. Filtereinheit nach einem der Ansprüche 1 bis 4, **dadurch gekennzeichnet, dass** B von einem Monomer stammt, das ausgewählt ist aus der Gruppe, bestehend aus einem Alkylacrylat, einem Alkylmethacrylat und einem Vinylalkoxid.

6. Filtereinheit nach einem der Ansprüche 1 bis 5, **dadurch gekennzeichnet, dass** die Einheit B eine der zwei folgenden Strukturen besitzt: 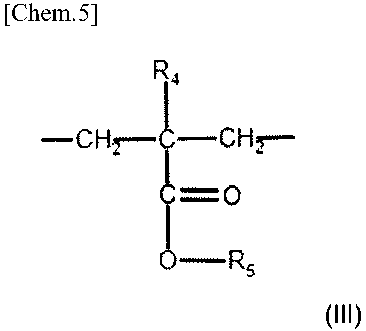 wobei R4 ausgewählt ist aus der Gruppe, bestehend aus einem Wasserstoffatom und der Methylgruppierung, und R5 eine lineare oder verzweigte C1-C6-Alkylgruppierung ist, oder 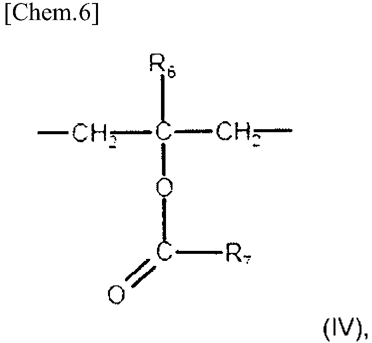 wobei R6 ausgewählt ist aus der Gruppe, bestehend aus einem Wasserstoffatom und der Methylgruppierung, und R7 eine C1-C6-Alkylgruppierung ist.

7. Filtereinheit nach einem der Ansprüche 1 bis 6, **dadurch gekennzeichnet, dass** die Einheit B von dem Methylmethacrylat oder dem Vinylacetat stammt.

8. Filtereinheit nach einem der Ansprüche 1 bis 7, **dadurch gekennzeichnet, dass** die zahlenmittlere Molmasse des Polymers zwischen 5000 g/mol und 40.000 g/mol liegt.

9. Filtereinheit nach einem der Ansprüche 1 bis 8, **dadurch gekennzeichnet, dass** die zahlenmittlere Molmasse des Copolymers zwischen 15.000 g/mol und 115.000 g/mol liegt.

10. Filtereinheit nach einem der Ansprüche 1 bis 9, **dadurch gekennzeichnet, dass** das Polymer einen Polymolekularitätsindex besitzt, der zwischen 1 und 6 liegt.

11. Filtereinheit nach einem der Ansprüche 1 bis 10, **dadurch gekennzeichnet, dass** die Menge an Copolymer, das auf das Leukozytendepletionsmedium aufgebracht ist, zwischen 1 und 10 mg/g Leukozytendepletionsmedium liegt.

12. Filtereinheit nach einem der Ansprüche 1 bis 11, **dadurch gekennzeichnet, dass** das Leukozytendepletionsmedium Fasern aus Polybutylenterephthalat oder Polyethylenterephthalat umfasst.

13. Filtereinheit nach einem der Ansprüche 1 bis 12, **dadurch gekennzeichnet, dass** das Leukozytendepletionsmedium aus mindestens einer Vliesschicht gebildet ist.

14. Filtereinheit nach Anspruch 13, **dadurch gekennzeichnet, dass** der mittlere Porendurchmesser der Vliesschicht zwischen 3 und 15 µm liegt.

15. Beutelsystem (10) für die Leukozytendepletion eines Fluids, das Blutplättchen enthält, **dadurch gekennzeichnet, dass** es Folgendes umfasst:
- eine Filtereinheit nach einem der Ansprüche 1 bis 14 und
- einen Beutel (11) zum Auffangen des Filtrats, wobei der Beutel über einen Stutzen (12) an eine Auslassöffnung (3) der Filtereinheit (1) angeschlossen ist.

16. Anordnung, umfassend einerseits ein Beutelsystem (10) nach Anspruch 15, wobei die Filtereinheit (1) eine weiche Hülle, und andererseits ein starres Etui (21), das dazu bestimmt ist, die Filtereinheit zu enthalten, umfasst, wobei das Etui (21) konfiguriert ist, um ein Aufblähen der Filtereinheit (1) während des Filterns zu verhindern.

## Claims

1. A filtration unit (1) for allowing the selective leukodepletion of a fluid containing blood platelets comprising an outer housing provided with at least one inlet port (2) and at least one outlet port (3), the housing enclosing a porous element (4) interposed between said ports, said porous element enclosing a leukodepletion medium impregnated with a copolymer, said filtration unit being **characterised in that** said copolymer has the general formula -(A)m-(B)n- (I), in which A is a unit derived from an alkoxyalkyl acrylate or alkoxyalkyl methacrylate monomer, B is a unit derived from a hydrophobic polymerisable monomer, and m and n are integers of which the sum m+n is equal to 100, m being an integer greater than 80.

2. The filtration unit according to claim 1, **characterised in that** the unit A has the following structure: 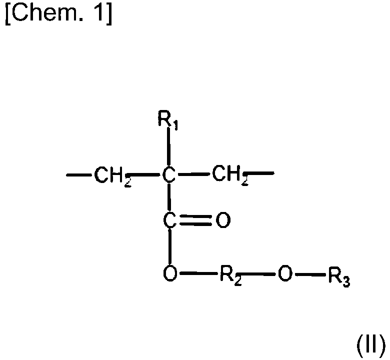 in which R1 is chosen from the group consisting of a hydrogen atom or the methyl group, R2 is a linear or branched C1-C4 alkylene bond and R3 is a linear or branched C1-C4 alkyl group.

3. The filtration unit according to claim 1 or 2, **characterised in that** the unit A is derived from methoxyethyl acrylate.

4. The filtration unit according to any one of claims 1 to 3, **characterised in that** m is an integer between 90 and 96.

5. The filtration unit according to any one of claims 1 to 4, **characterised in that** B derives from a monomer chosen from the group consisting of an alkyl acrylate, an alkyl methacrylate and a vinyl alkylate.

6. The filtration unit according to any one of claims 1 to 5, **characterised in that** the unit B has one of the following two structures: 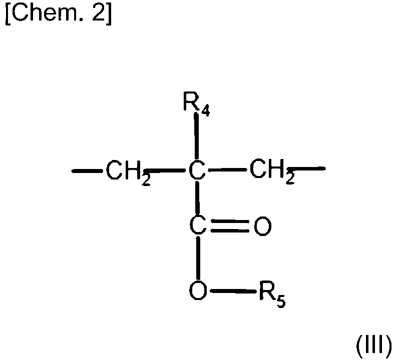 in which R4 is chosen from the group consisting of a hydrogen atom and the methyl group and R5 is a linear or branched C1-C6 alkyl group, or 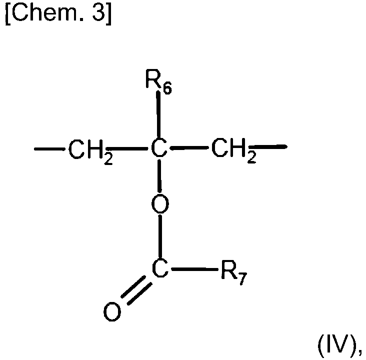 in which R6 is chosen from the group consisting of a hydrogen atom and the methyl group and R7 is a C1-C6 alkyl group.

7. The filtration unit according to any one of claims 1 to 6, **characterised in that** the unit B is derived from methyl methacrylate or vinyl acetate.

8. The filtration unit according to any one of claims 1 to 7, **characterised in that** the number-average molar mass of said polymer is between 5,000 g/mol and 40,000 g/mol.

9. The filtration unit according to any one of claims 1 to 8, **characterised in that** the weight-average molar mass of said copolymer is between 15,000 g/mol and 115,000 g/mol.

10. The filtration unit according to any one of claims 1 to 9, **characterised in that** the polymer has a polymolecularity index of between 1 and 6.

11. The filtration unit according to any one of claims 1 to 10, **characterised in that** the quantity of copolymer deposited on the leukodepletion medium is between 1 and 10 mg/g of leukodepletion medium.

12. The filtration unit according to any one of claims 1 to 11, **characterised in that** the leukodepletion medium comprises polybutylene terephthalate or polyethylene terephthalate fibres.

13. The filtration unit according to any one of claims 1 to 12, **characterised in that** the leukodepletion medium is formed by at least one layer of non-woven fabric.

14. The filtration unit according to claim 13, **characterised in that** the average pore diameter of the non-woven layer is between 3 and 15 µm.

15. A bag system (10) for the leukodepletion of a fluid containing blood platelets, **characterised in that** it comprises:
- a filtration unit according to any one of claims 1 to 14, and
- a bag (11) for collecting the filtrate, said bag being connected, by means of a tube (12), to an outlet port (3) of the filtration unit (1).

16. An assembly comprising, on the one hand, a bag system (10) according to claim 15, in which the filtration unit (1) comprises a flexible housing and, on the other hand, a rigid case (21) intended to contain said filtration unit, said case (21) being configured to prevent the filtration unit (1) from swelling during the filtration.
